# EUROPEAN PATENT SPECIFICATION

(11) **EP 3 519 069 B1**
(45) Date of publication and mention of the grant of the patent: **26.05.2021**
(21) Application number: 17856901.8
(22) Date of filing: 27.06.2017
(51) Int. Cl.: B01D 3/14, C07D 313/04, C08G 63/08

(54) **PROCESS FOR PROCESSING OF A PRODUCT STREAM RESULTING FROM A CAPROLACTONE PRODUCTION**
VERFAHREN ZUR VERARBEITUNG EINES PRODUKTSTROMS AUS EINER CAPROLACTON-PRODUKTION
PROCÉDÉ DE TRAITEMENT D'UN FLUX DE PRODUIT RÉSULTANT D'UNE PRODUCTION DE CAPROLACTONE

(30) Priority: 28.09.2016 SE 1630234
(43) Date of publication of application: 07.08.2019
(73) Proprietor: Ingevity UK Ltd, Warrington Cheshire WA4 6HA (GB)
(72) Inventor: PAJALIC, Oleg, 291 06 Kristianstad (SE); BERGGREN, John, 252 22 Helsingborg (SE); SKOGESTAD, Sigurd, 7048 Trondheim (NO); LE, Quang-Koha, Ho Chi Minh City 700000 (VN)
(74) Representative: Hoffmann Eitle
(86) International application number: PCT/SE2017/050707
(87) International publication number: WO 2018/063049

(56) References cited:
- DE-A1- 10 102 168
- DE-A1- 10 308 489
- DE-A1- 19 607 954
- US-A1- 2004 087 804
- US-A1- 2011 092 749
- US-B2- 7 329 330

## Description

The present invention refers to a process for processing of a product stream (a) comprising acetic acid, unreacted cyclohexanone, reaction water and organic heavy ends, said product stream (a) being yielded in a caprolactone production wherein peracetic acid is generated from acetic acid and hydrogen peroxide according to reaction scheme (I) below 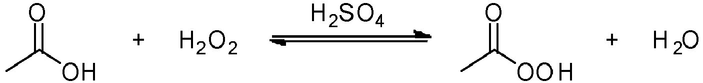 and wherein yielded peracetic acid is subjected to a Baeyer-Villiger reaction with cyclohexanone according to reaction scheme (II) below 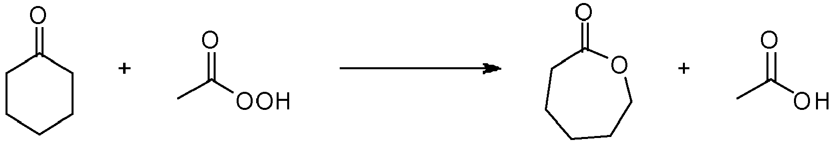 yielding a reaction mixture comprising caprolactone, acetic acid, unreacted cyclohexanone, reaction water and heavy organic ends, which reaction mixture is further processed yielding purified caprolactone and at least one product stream (a) comprising acetic acid, unreacted cyclohexanone, reaction water and heavy ends A conventional process for the production of caprolactone is disclosed in US2004/0087804A1.

It is an object of the present invention to provide an improved, in particular a more economical process for obtaining pure products from said product stream (a). It has been found that this object is achieved by a process comprising a divided wall column (1) for separating by distillation said product stream (a) obtained in said caprolactone process. Investment costs can, by the present and claimed invention, be reduced with as much as 20% and heating, such as steam, costs by 20-30% compared with conventionally and typically used equipment comprising for instance at least one primary and one secondary distillation column. The present invention is accordingly directed to a process for processing of a said product stream (a), whereby said process in embodiments of the present invention comprises/involves as main equipment a divided wall column (1) comprising an upper section (2), a bottom section (3), a feed section (4) and a take-off section (5), a separator (9), a distillation column (20) and optionally a distillation column (15). Sections (4) and (5) comprise in especially preferred embodiments each at least one rectification and at least one stripping section. Divided wall columns, that is distillation columns having vertical dividing walls preventing cross mixing of liquid streams and/or vapor streams in certain regions, are known for the separation of multicomponent mixtures by distillation. Divided wall columns typically have a dividing wall which divides the interior of the column into the following regions an upper section, a bottom section and a feed section and a take-off section. Mixture to be separated are introduced in the region of the feed section, high boiling fractions are removed from the bottom of the column and low boiling fractions are removed via the top of the column.

Said product stream (a), which typically comprises by weight for instance 45-65% of acetic acid, 30-40% of cyclohexanone, 1-15% of water and 0-1% of heavy ends, is in preferred embodiments of the present invention fed to the feed section (4) via an inlet (6) in the middle of said feed section (4) and said product stream (a) is in said feed section (4) by distillation separated in a water/acetic acid fraction (b) and a cyclohexanone/heavy end fraction (c), which typically comprises for instance more than 95% by weight of cyclohexanone.

The water/acetic acid fraction (b) is via an outlet (25) in said upper section (2) and an inlet (26) fed to a separator (9). Said acetic acid/water fraction is a homogeneous azeotrope which in presence of at least one entrainer is broken and in said separator (9) separated in an organic phase (d) comprising acetic acid and a minor amount of water and a water phase (e) comprising water and a minor amount of acetic acid. Entrainer(s) can be added for instance from a tank (24) via an inlet (10) arranged at said separator (9). The organic phase (d) is subsequently fed to said divided wall column (1) through an outlet (11) in said separator (9) and an inlet (12) in said upper section (2) and further evaporated yielding high concentrated acetic acid (f) typically having an acetic acid percentage of more than 95%, such as at least 98%, by weight, which is taken out via an outlet (13) in the middle of said take-off section (5) and preferably recycled to said peracetic acid generation or alternatively fed to for instance a storage tank for later use, and the water phase (e) is either dumped via an outlet (14) in said separator (9) or preferably via said outlet (14) fed to a distillation column (15) via an inlet (16) and further processed whereby water and said minor amount of acetic acid are separated in an acidic fraction (h) and a water fraction (i) consisting essentially of water. The acidic fraction (h) is preferably recycled, for recovery of remaining acetic acid, to said separator (9) via an outlet (17) arranged for instance at the top of said column (15) and an inlet (23) on said separator (9) and said fraction (i) can be dumped, sent to destruction or sent for further processing via an outlet (18) at the bottom of said column (15).

The cyclohexanone/heavy end fraction (c) is in preferred embodiments, via an outlet (7) in said bottom section (3) and via an inlet (19) fed to a distillation column (20) wherein cyclohexanone and heavy ends are separated in a purified cyclohexanone fraction (g) and a heavy end fraction (j). In said embodiments, the purified cyclohexanone fraction (g), typically having a cyclohexanone percentage of at least 98%, such as more than 99%, by weight is via an outlet (21) preferably recycled to said Baeyer-Villiger reaction or alternatively fed to for instance a storage tank for later use and the heavy end fraction (j) is via an outlet (22) either sent to destruction or to further processing.

The divided wall column (1) is, in preferred embodiments of the present invention, operated at a temperature of for instance 80-180°C, such as 100-160°C, and at a pressure of 0.5-1.5 bar, such as 1.0-1.3 bar. Said separator (9) is, in likewise preferred embodiments, a liquid phase separator operated at a temperature of for instance 35-45°C and at a pressure of for instance 0.8-1.2 bar. Column (20) is preferably operated at a temperature of for instance 50-150°C and at a pressure of for instance 0.05-0.2 bar, and said optional column (15) is in preferred embodiments distillation column heated by a steam generator (27) and operated at a temperature of for instance 95-105°C and at atmospheric pressure.

Said entrainer is in especially preferred embodiments of the present invention a C₁-C₆ alkyl acetate, such as methyl acetate, ethyl acetate, propyl acetate, isopropyl acetate, butyl acetate and/or isobutyl acetate. Further possible entrainers include aromatic and aliphatic hydrocarbons, such as toluene and hexane, and ethers, such as methyl tert.butyl ether. Entrainers for distillation of acetic acid/water systems are disclosed and discussed in for instance "Entrainer for batch distillation of acetic acid - water system" S.V. Gadekar et al., Journal of Scientific & Industrial Research, vol. 68, Oct. 2009, pp 871-875.

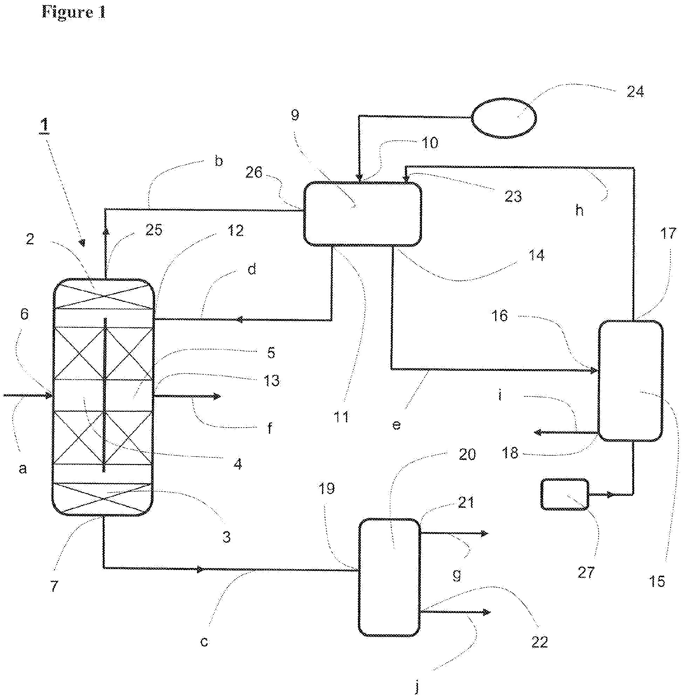

## Claims

1. A process for processing of a product stream (a) comprising acetic acid, unreacted cyclohexanone, reaction water and organic heavy ends, which product stream (a) is yielded in a caprolactone production wherein peracetic acid is generated from acetic acid and hydrogen peroxide and wherein said peracetic acid is subjected to a Baeyer-Villiger reaction with cyclohexanone **characterised in, that** said process comprises and involves a divided wall column (1) having an upper section (2), a bottom section (3), a feed section (4) and a take-off section (5), that said product stream (a) is fed to said feed section (4) via an inlet (6) in the middle of said feed section (4), that said product stream (a) in said feed section (4) by distillation is separated in a water/acetic acid fraction (b) and a cyclohexanone/heavy end fraction (c), which water/acetic acid fraction (b) via an outlet (25) in said upper section (2) and an inlet (26) is fed to a separator (9), that said acetic acid/water fraction is a homogeneous azeotrope which in presence of at least one entrainer is broken and in said separator (9) separated in an organic phase (d) comprising acetic acid and a minor amount of water and a water phase (e) comprising water and a minor amount of acetic acid, that said organic phase (d) comprising acetic acid and a minor amount of water is fed to said divided wall column (1) through an outlet (11) in said separator (9) and an inlet (12) in said upper section (2) and further evaporated yielding high concentrated acetic acid (f) being taken out via an outlet (13) in the middle of said take-off section (5) and either recycled to said peracetic acid generation or fed to a storage tank, and the water phase (e) comprising water and a minor amount of acetic acid is either dumped via an outlet (14) or via said outlet (14) in said separator (9) fed to a distillation column (15) via an inlet (16) and further processed whereby water and said minor amount of acetic acid is separated in an acidic fraction (h) being recycled to said separator (9) via an outlet (17) arranged at said column (15) and an inlet (23) on said separator (9) and a water fraction (i) being dumped or sent for further processing via an outlet (18) at the bottom of said column (15), and that said cyclohexanone/heavy end fraction (c), via an outlet (7) in said bottom section (3) and via an inlet (19) is fed to a distillation column (20) wherein cyclohexanone and heavy ends are separated in a purified cyclohexanone fraction (g) which via an outlet (21) either is recycled to said Baeyer-Villiger reaction or fed to a storage tank and a heavy end fraction (j) which via an outlet (22) is either dumped or sent to destruction or to further processing.

2. The process according to claim 1 **characterised in, that** sections (4) and (5) comprise each at least one rectification and at least one stripping section.

3. The process according to claim 1 or 2 **characterised in, that** at least one entrainer is added from a tank (24) via an inlet (10) arranged at said separator (9).

4. The process according to any of the claims 1-3 **characterised** i**n**, that said divided wall column (1) is operated at a temperature of 80-180°C and a pressure of 0.5-1.5 bar.

5. The process according to any of the claims 1-4 **characterised in, that** said divided wall column (1) is operated at a temperature of 100-160°C and a pressure of 1.0-1.3 bar.

6. The process according to any of the claims 1-5 **characterised** i**n**, that said separator (9) is a liquid phase separator operated at a temperature of 35-45°C and a pressure of 0.8-1.2 bar.

7. The process according to any of the claims 1-6 **characterised in, that** said column (20) is a distillation column operated at a temperature of 50-150°C and a pressure of 0.05-0.2 bar.

8. The process according to any of the claims 1-7 **characterised in, that** said column (15) is a distillation column heated by a steam generator (27) and operated at a temperature of 95-105°C and at atmospheric pressure.

9. The process according to any of the claims 1-8 **characterised in, that** said entrainer is a C₁-C₆ alkyl acetate.

10. The process according to any of the claims 1-9 **characterised in, that** said entrainer is ethyl acetate, propyl acetate, isopropyl acetate, butyl acetate and/or isobutyl acetate.

11. The process according to any of the claims 1-10 **characterised in, that** said product stream (a) comprises by weight 45-65% of acetic acid, 30-40% of cyclohexanone, 1-15% of water and 0-1% of heavy ends.

12. The process according to any of the claims 1-11 **characterised in, that** said cyclohexanone/heavy end fraction (c) comprises more than 95% by weight of cyclohexanone.

13. The process according to any of the claims 1-12 **characterised in, that** said high concentrated acetic acid (f) has an acetic acid percentage by weight of at least 95%.

14. The process according to any of the claims 1-13 **characterised in, that** said high concentrated acetic acid (f) via said outlet (13) is recycled to said peracetic generation.

15. The process according to any of the claims 1-14 **characterised in, that** said purified cyclohexanone (g) has a cyclohexanone content of more than 98% by weight and that said purified cyclohexanone (g) via said outlet (21) is recycled to said Baeyer-Villiger reaction.

## Patentansprüche

1. Verfahren zur Herstellung eines Produktstroms (a), umfassend Essigsäure, nicht umgesetztes Cyclohexanon, Reaktionswasser und organische Nachläufe, wobei der Produktstrom (a) bei der Herstellung von Caprolacton erhalten wird, worin Peressigsäure aus Essigsäure und Wasserstoffperoxid erzeugt wird und worin die Peressigsäure in einer Baeyer-Villiger-Reaktion mit Cyclohexanon umgesetzt wird, **dadurch gekennzeichnet, dass** das Verfahren eine Trennwandkolonne (1) mit einem oberen Abschnitt (2), einem unteren Abschnitt (3), einem Zufuhrabschnitt (4) und einem Entnahmeabschnitt (5) umfasst und einschließt, dass der Produktstrom (a) dem Zufuhrabschnitt (4) über einen Einlass (6) in der Mitte des Zufuhrabschnitts (4) zugeführt wird, dass der Produktstrom (a) in dem Zufuhrabschnitt (4) mittels Destillation in eine Wasser-/Essigsäurefraktion (b) und eine Cyclohexanon-/Nachlauffraktion (c) aufgetrennt wird, wobei die Wasser-/Essigsäurefraktion (b) über einen Auslass (25) in dem oberen Abschnitt (2) und einen Einlass (26) einem Abscheider (9) zugeführt wird, dass die Essigsäure-/Wasserfraktion ein homogenes Azeotrop ist, das in Gegenwart mindestens eines Schleppmittels aufgebrochen wird und in dem Abscheider (9) in eine organische Phase (d), die Essigsäure und eine geringe Menge an Wasser umfasst, und eine Wasserphase (e), die Wasser und eine geringe Menge an Essigsäure umfasst, aufgetrennt wird, dass die organische Phase (d), die Essigsäure und eine geringe Menge an Wasser umfasst, der Trennwandkolonne (1) durch einen Auslass (11) in dem Abscheider (9) und einen Einlass (12) in dem oberen Abschnitt (2) zugeführt wird und ferner verdampft wird, wobei hochkonzentrierte Essigsäure (f) erhalten wird, die über einen Auslass (13) in der Mitte des Entnahmeabschnitts (5) entnommen wird und entweder der Erzeugung von Peressigsäure zurückgeführt wird oder einem Lagerbehälter zugeführt wird, und die Wasserphase (e), die Wasser und eine geringe Menge an Essigsäure umfasst, entweder über einen Auslass (14) abgelassen wird oder über den Auslass (14) in dem Abscheider (9) einer Destillationskolonne (15) über einen Einlass (16) zugeführt wird und ferner aufbereitet wird, wodurch Wasser und die geringe Menge an Essigsäure in eine saure Fraktion (h), die dem Abscheider (9) über einen an der Kolonne (15) angeordneten Auslass (17) und einen Einlass (23) an dem Abscheider (9) zurückgeführt wird, und eine Wasserfraktion (i), die abgelassen wird oder zur weiteren Aufbereitung über einen Auslass (18) am unteren Abschnitt der Kolonne (15) befördert wird, aufgetrennt werden, und dass die Cyclohexanon-/Nachlauffraktion (c) über einen Auslass (7) in dem unteren Abschnitt (3) und über einen Einlass (19) einer Destillationskolonne (20) zugeführt wird, worin Cyclohexanon und der Nachlauf in eine aufgereinigte Cyclohexanonfraktion (g), die über einen Auslass (21) entweder der Baeyer-Villiger-Reaktion zurückgeführt wird oder einem Lagerbehälter zugeführt wird, und eine Nachlauffraktion (j), die über einen Auslass (22) entweder abgelassen wird oder zur Beseitigung oder weiteren Aufarbeitung befördert wird, aufgetrennt werden.

2. Verfahren gemäß Anspruch 1, **dadurch gekennzeichnet, dass** die Abschnitte (4) und (5) jeweils mindestens einen Abschnitt zur Rektifikation und einen Abschnitt zur Strippung umfassen.

3. Verfahren gemäß Anspruch 1 oder 2, **dadurch gekennzeichnet, dass** das mindestens eine Schleppmittel aus einem Behälter (24) über einen an dem Abscheider (9) angeordneten Einlass (10) zugeführt wird.

4. Verfahren gemäß einem der Ansprüche 1-3, **dadurch gekennzeichnet, dass** die Trennwandkolonne (1) bei einer Temperatur von 80-180°C und einem Druck von 0,5-1,5 bar betrieben wird.

5. Verfahren gemäß einem der Ansprüche 1-4, **dadurch gekennzeichnet, dass** die Trennwandkolonne (1) bei einer Temperatur von 100-160°C und einem Druck von 1,0-1,3 bar betrieben wird.

6. Verfahren gemäß einem der Ansprüche 1-5, **dadurch gekennzeichnet, dass** der Abscheider (9) ein Flüssigphasenabscheider ist, der bei einer Temperatur von 35-45°C und einem Druck von 0,8-1,2 bar betrieben wird.

7. Verfahren gemäß einem der Ansprüche 1-6, **dadurch gekennzeichnet, dass** die Kolonne (20) eine Destillationskolonne ist, die bei einer Temperatur von 50-150°C und einem Druck von 0,05-0,2 bar betrieben wird.

8. Verfahren gemäß einem der Ansprüche 1-7, **dadurch gekennzeichnet, dass** die Kolonne (15) eine Destillationskolonne ist, die durch einen Dampferzeuger (27) erwärmt wird und bei einer Temperatur von 95-105°C und Atmosphärendruck betrieben wird.

9. Verfahren gemäß einem der Ansprüche 1-8, **dadurch gekennzeichnet, dass** das Schleppmittel ein C₁-C₆-Alkylacetat ist.

10. Verfahren gemäß einem der Ansprüche 1-9, **dadurch gekennzeichnet, dass** das Schleppmittel Ethylacetat, Propylacetat, Isopropylacetat, Butylacetat und/oder Isobutylacetat ist.

11. Verfahren gemäß einem der Ansprüche 1-10, **dadurch gekennzeichnet, dass** der Produktstrom (a) 45-65 Gew.-% Essigsäure, 30-40 Gew.-% Cyclohexanon, 1-15 Gew.-% Wasser und 0-1% Nachlauf umfasst.

12. Verfahren gemäß einem der Ansprüche 1-11, **dadurch gekennzeichnet, dass** die Cyclohexanon-/Nachlauffraktion (c) mehr als 95 Gew.-% Cyclohexanon umfasst.

13. Verfahren gemäß einem der Ansprüche 1-12, **dadurch gekennzeichnet, dass** die hochkonzentrierte Essigsäure (f) einen Essigsäureanteil von mindestens 95 Gew.-% aufweist.

14. Verfahren gemäß einem der Ansprüche 1-13, **dadurch gekennzeichnet, dass** die hochkonzentrierte Essigsäure (f) über den Auslass (13) der Erzeugung von Peressigsäure zurückgeführt wird.

15. Verfahren gemäß einem der Ansprüche 1-14, **dadurch gekennzeichnet, dass** das aufgereinigte Cyclohexanon (g) einen Cyclohexanongehalt von mehr als 98 Gew.-% aufweist und dass das aufgereinigte Cyclohexanon (g) über den Auslass (21) der Baeyer-Villiger-Reaktion zurückgeführt wird.

## Revendications

1. Processus de traitement d'un flux de produit (a) comprenant de l'acide acétique, de la cyclohexanone n'ayant pas réagi, de l'eau de réaction et des extrémités lourdes organiques, lequel flux de produit (a) est produit dans une production de caprolactone dans laquelle l'acide peracétique est généré à partir d'acide acétique et de peroxyde d'hydrogène et dans laquelle ledit acide peracétique est soumis à une réaction de Baeyer-Villiger avec de la cyclohexanone, **caractérisé en ce que** ledit processus comprend et implique une colonne de paroi divisée (1) présentant une section supérieure (2), une section inférieure (3), une section d'alimentation (4) et une section de décollage (5), **en ce que** ledit flux de produit (a) est alimenté vers ladite section d'alimentation (4) par l'intermédiaire d'une entrée (6) au milieu de ladite section d'alimentation (4), **en ce que** ledit flux de produit (a) dans ladite section d'alimentation (4) par distillation est séparé en une fraction eau/acide acétique (b) et une fraction cyclohexanone/extrémité lourde (c), laquelle fraction eau/acide acétique (b) par l'intermédiaire d'une sortie (25) dans ladite section supérieure (2) et d'une entrée (26) est alimentée vers un séparateur (9), **en ce que** ladite fraction acide acétique/eau est un azéotrope homogène qui, en présence d'au moins un agent de séparation, est brisé et dans ledit séparateur (9) séparé en une phase organique (d) comprenant de l'acide acétique et une petite quantité d'eau et une phase aqueuse (e) comprenant de l'eau et une petite quantité d'acide acétique, **en ce que** ladite phase organique (d) comprenant de l'acide acétique et une petite quantité d'eau est alimentée vers ladite colonne de paroi divisée (1) à travers une sortie (11) dans ledit séparateur (9) et une entrée (12) dans ladite section supérieure (2) et de l'acide acétique de production à concentration élevée évaporé (f) en outre étant retiré par l'intermédiaire d'une sortie (13) au milieu de ladite section de décollage (5) et soit recyclé vers ladite génération d'acide peracétique soit alimenté vers un réservoir de stockage, et la phase aqueuse (e) comprenant de l'eau et une petite quantité d'acide acétique est soit jetée par l'intermédiaire d'une sortie (14) soit par l'intermédiaire de ladite sortie (14) dans ledit séparateur (9) alimentée vers une colonne de distillation (15) par l'intermédiaire d'une entrée (16) et en outre traitée selon lequel de l'eau et ladite petite quantité d'acide acétique sont séparées en une fraction acide (h) étant recyclée vers ledit séparateur (9) par l'intermédiaire d'une sortie (17) agencée au niveau de ladite colonne (15) et d'une entrée (23) sur ledit séparateur (9) et une fraction aqueuse (i) étant jetée ou envoyée pour un traitement supplémentaire par l'intermédiaire d'une sortie (18) dans le fond de ladite colonne (15), et **en ce que** ladite fraction cyclohexanone/extrémité lourde (c), par l'intermédiaire d'une sortie (7) dans ladite section inférieure (3) et par l'intermédiaire d'une entrée (19), est alimentée vers une colonne de distillation (20) dans laquelle de la cyclohexanone et des extrémités lourdes sont séparées dans une fraction de cyclohexanone purifiée (g) qui, par l'intermédiaire d'une sortie (21), est soit recyclée vers ladite réaction de Baeyer-Villiger soit alimentée vers un réservoir de stockage et une fraction d'extrémité lourde (j) qui, par l'intermédiaire d'une sortie (22) est soit jetée soit envoyée en destruction ou à un traitement supplémentaire.

2. Processus selon la revendication 1, **caractérisé en ce que** des sections (4) et (5) comprennent chacune au moins une rectification et au moins une section de fractionnement.

3. Processus selon la revendication 1 ou 2, **caractérisé en ce qu'**au moins un agent de séparation est ajouté depuis un réservoir (24) par l'intermédiaire d'une entrée (10) agencée au niveau dudit séparateur (9).

4. Processus selon l'une quelconque des revendications 1-3, **caractérisé en ce que** ladite colonne de paroi divisée (1) fonctionne à une température de 80 à 180 °C et une pression de 0,5 à 1,5 bar.

5. Processus selon l'une quelconque des revendications 1-4, **caractérisé en ce que** ladite colonne de paroi divisée (1) fonctionne à une température de 100 à 160 °C et une pression de 1,0 à 1,3 bar.

6. Processus selon l'une quelconque des revendications 1-5, **caractérisé en ce que** ledit séparateur (9) est un séparateur de phase liquide fonctionnant à une température de 35 à 45 °C et une pression de 0,8 à 1,2 bar.

7. Processus selon l'une quelconque des revendications 1-6, **caractérisé en ce que** ladite colonne (20) est une colonne de distillation fonctionnant à une température de 50 à 150 °C et une pression de 0,05 à 0,2 bar.

8. Processus selon l'une quelconque des revendications 1-7, **caractérisé en ce que** ladite colonne (15) est une colonne de distillation chauffée par un générateur de vapeur (27) et fonctionnant à une température de 95 à 105 °C et à pression atmosphérique.

9. Processus selon l'une quelconque des revendications 1-8, **caractérisé en ce que** ledit agent de séparation est un acétate d'alkyle en C₁-C₆.

10. Processus selon l'une quelconque des revendications 1-9, **caractérisé en ce que** ledit agent de séparation est un acétate d'éthyle, un acétate de propyle, un acétate d'isopropyle, un acétate de butyle et/ou un acétate d'isobutyle.

11. Processus selon l'une quelconque des revendications 1-10, **caractérisé en ce que** ledit flux de produit (a) comprend, en poids, de 45 à 65 % d'acide acétique, de 30 à 40 % de cyclohexanone, de 1 à 15 % d'eau et de 0 à 1 % d'extrémités lourdes.

12. Processus selon l'une quelconque des revendications 1-11, **caractérisé en ce que** ladite fraction cyclohexanone/extrémité lourde (c) comprend plus de 95 % en poids de cyclohexanone.

13. Processus selon l'une quelconque des revendications 1-12, **caractérisé en ce que** ledit acide acétique à concentration élevée (f) présente un pourcentage d'acide acétique en poids d'au moins 95 %.

14. Processus selon l'une quelconque des revendications 1-13, **caractérisé en ce que** ledit acide acétique à concentration élevée (f) par l'intermédiaire de ladite sortie (13) est recyclé vers ladite génération d'acide peracétique.

15. Processus selon l'une quelconque des revendications 1-14, **caractérisé en ce que** ladite cyclohexanone purifiée (g) présente une teneur en cyclohexanone de plus de 98 % en poids et **en ce que** ladite cyclohexanone purifiée (g) par l'intermédiaire de ladite sortie (21) est recyclée vers ladite réaction de Baeyer-Villiger.
